# EUROPEAN PATENT SPECIFICATION

(11) **EP 3 177 290 B1**
(45) Date of publication and mention of the grant of the patent: **20.06.2018**
(21) Application number: 15722530.1
(22) Date of filing: 13.05.2015
(51) Int. Cl.: A61K 31/444, A61K 9/20, A61P 7/02

(54) **PHARMACEUTICAL COMPOSITIONS OF EDOXABAN**
PHARMAZEUTISCHE ZUSAMMENSETZUNGEN VON EDOXABAN
COMPOSITIONS PHARMACEUTIQUES D'EDOXABAN

(30) Priority: 06.08.2014 EP 14180078
(43) Date of publication of application: 14.06.2017
(73) Proprietor: Sandoz AG, 4056 Basel (CH)
(72) Inventor: RANEBURGER, Johannes, A-6250 Kundl (AT); FAROUK, Hossam, A-6250 Kundl (AT)
(74) Representative: Prüfer & Partner mbB Patentanwälte · Rechtsanwälte
(86) International application number: PCT/EP2015/060656
(87) International publication number: WO 2016/020080

(56) References cited:
- EP-A1- 2 444 087
- EP-A1- 2 548 556

## Description

### FIELD OF INDUSTRIAL APPLICABILITY

The present invention relates to pharmaceutical compositions of Edoxaban, processes for their preparation and their medical use.

### BACKGROUND OF THE DISCLOSURE

Edoxaban, N1-(5-chloropyridin-2-yl)-N2-((1S,2R,4S)-4-[(dimethylamino)carbonyl]-2-f[(5-methyl-4,5,6,7-tetrahydrothiazolo[5,4-c]pyridin-2-yl)carbonyl]aminolcyclohexyl)ethanediamide, which is represented by the following formula (I) 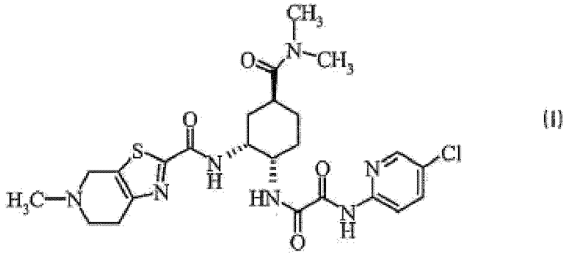 is known to exhibit an inhibitory effect on activated blood coagulation factor X and to be useful as a pharmaceutical drug, particularly for preventing and/or treating thrombosis or embolism. Edoxaban is a basic compound that exhibits favorable solubility in a strongly acidic aqueous solution, but reduced solubility in an aqueous solution having a pH in the neutral region (e.g., a neutral buffer). Therefore, a solid formulation containing Edoxaban or a pharmaceutically acceptable salt thereof as an active ingredient exhibits a poor dissolution property in an aqueous solution having a pH in the neutral region.

Methods for improving the dissolution rate of Edoxaban are known in the art. WO 2008/129846 A1 discloses compositions containing Edoxaban combined with a sugar alcohol and/or a water-swelling additive, as well as coating compositions containing Edoxaban. However many people have intolerance against sugar alcohols, such as sorbitol, and some coating agents, such as HPMC. WO 2010/147169 A1 discloses reducing the proportion of Edoxaban in the pharmaceutical composition (0.5 -10%), which however results in big dosage forms that are difficult to swallow and negatively impact patient compliance to the therapy. Alternatively, it is known from WO 2011/115067 A1 that the dissolution property in the neutral region of a solid formulation containing Edoxaban is improved by keeping the maximum water content of granulated material containing Edoxaban during granulation at 10% or less. However this process works only with formulations containing a sugar alcohol and/or a water-swelling additive, which suffer from the above mentioned drawbacks. WO 2013/022059 A1 discloses that the addition of an acid to formulations of Edoxaban seems to improve its dissolutions properties; however the examples relate to compositions containing very high amounts of acids, which thus reduce the drug load, as well as specific fillers, such as mannitol and pregelatinized starch. WO 2013/026553 A1 discloses effervescent formulations of Edoxaban comprising a carbon dioxide forming agent, such as sodium bicarbonate, which need special precautions regarding manufacturing and packaging to prevent contact with moisture.

It turned out that prior art pharmaceutical formulations comprising Edoxaban being suitable for oral administration are still improvable with regards to dissolution rate and bioavailability. Further, due to the intolerance of many people to sugar alcohols, such as sorbitol, the use of sugar alcohols should be avoided.

Hence, it is an object of the present invention to overcome the above-mentioned disadvantages. It is a further object of the invention to provide a dosage form being free of sugar alcohols. It is a further object of the invention to provide the active agent in a formulation that possesses a superior and pH independent dissolution rate and bioavailability, as well as at the same time superior storage stability. It is a further object of the present invention to provide with good flowability, easy processing and superior content uniformity of the active agent.

### SUMMARY OF THE DISCLOSURE

The present disclosure provides a pharmaceutical composition comprising Edoxaban, or a pharmaceutically acceptable salt thereof, a water soluble vinylpyrrolidone polymer selected from the group consisting of povidone and copovidone, and a cellulose ether, and not pharmaceutically acceptable salt thereof, a water soluble vinylpyrrolidone polymer selected from the group consisting of povidone and copovidone, and a cellulose ether, and not comprising a sugar alcohol, a process for its preparation, a dosage form comprising such composition, and the use of said pharmaceutical composition and dosage form as a medicament.

### DETAILED DESCRIPTION OF THE DISCLOSURE

### Definitions

As used herein "povidone" refers to polyvinylpyrrolidone, a water-soluble polymer made from the monomer N-vinylpyrrolidone.

As used herein "copovidone" refers to a water-soluble vinylpyrrolidone-vinyl acetate copolymer that contains the two components in a ratio of about 6:4.

As used herein "cellulose ether" refers to derivatives of cellulose that are formed via partial of complete substitution of the hydrogen atoms of the hydroxyl groups of cellulose. Such reaction is known as etherification.

As used herein "granulating" refers to the formation of relatively coarse or granular aggregate material as a powder by assembling and/or aggregating finer powder particles (agglomerate formation, or build-up granulation) and/or the formation of finer granules by breaking up coarser aggregates (disintegration, or break-down granulation). Granulation can conventionally mean wet or dry granulation.

### Pharmaceutical compositions

It has surprisingly been found that when formulating Edoxaban, or a pharmaceutically acceptable salt thereof, with two different binders, the first being a water soluble vinylpyrrolidone polymer selected from the group consisting of povidone and copovidone, and the second a cellulose ether, notwithstanding the absence of a sugar alcohol fast and complete dissolution is achieved independently of the pH of the solution and thanks to surface activation and intensive interaction on molecular level between the binders and the active ingredient. Moreover subsequent precipitation of the active ingredient is avoided. Particularly good results are obtained if the formulation is prepared by wet granulation, which enables a more intimate contact of Edoxaban with the two binders.

Edoxaban free base or, preferably, a pharmaceutically acceptable salt thereof may be used. More preferably, the pharmaceutically acceptable salt of Edoxaban is Edoxaban tosylate. Particularly preferred is Edoxaban tosylate monohydrate. Preferably, the pharmaceutical composition of the present invention comprises 5 to 80%, more preferably 8 to 50%, even more preferably 10 to 30% of Edoxaban, calculated as weight of the free base on the total weight of the composition.

Povidone is preferably selected from the group consisting of povidone K12, K15, K17, K25, K30, K60, K90 and K120. Copovidone is preferably selected from the group consisting of Kollidon VA 64, Plasdone S-630 and Luvixol VA. Preferably, the pharmaceutical composition of the present invention comprises 1 to 20%, more preferably 2 to 15%, even more preferably 3 to 10% of the water soluble vinylpyrrolidone polymer, calculated as weight of vinylpyrrolidone polymer on the total weight of the composition.

The cellulose ether is preferably selected from the group consisting of hydroxypropyl methylcellulose (HPMC), hydroxypropyl cellulose (HPC), ethylcellulose (EC), hydroxyethyl cellulose (HEC), carboxymethyl cellulose (CMC) and methylcellulose (MC), preferably hydroxypropyl methylcellulose, hydroxypropyl cellulose, ethyl cellulose or hydroxyethyl cellulose, most preferably hydroxypropyl methylcellulose. Preferably, the pharmaceutical composition of the present invention comprises 1 to 20%, more preferably 2 to 15%, even more preferably 3 to 10% of the cellulose ether, calculated as weight of the cellulose ether on the total weight of the composition.

The pharmaceutical composition of the invention can preferably contain further inactive ingredients, preferably selected from the group consisting of fillers, disintegrants and lubricants.

The term "fillers" generally refers to substances which serve, for example, to form the body of the tablet in the case of tablets with small amounts of active agent. This means that fillers "dilute" the active agents in order to produce an adequate dosage form mixture. The normal purpose of fillers, therefore, is to obtain a suitable dosage form size, preferably a suitable tablet size. Examples of suitable fillers are microcrystalline cellulose, dibasic calcium phosphate, lactose, talcum, calcium phosphate, magnesium carbonate, magnesium oxide, calcium sulphate, saccharose, monosaccharides, such as glucose, maltodextrin, dextrates, dextrin. Preferred fillers are microcrystalline cellulose, dibasic calcium phosphate and lactose monohydrate.

Disintegrants expand and dissolve when wet causing the pharmaceutical composition to break apart in the digestive tract, releasing the active ingredients for absorption. They ensure that when the pharmaceutical composition is in contact with water, it rapidly breaks down into smaller fragments, facilitating dissolution. Examples of suitable disintegrants include crosslinked polymers, starches and modified starches. Preferred disintegrants are crosslinked polyvinylpyrrolidone (crospovidone), crosslinked sodium carboxymethyl cellulose (croscarmellose sodium), corn starch and sodium starch glycolate.

Lubricants are generally used in order to reduce sliding friction. Suitable lubricants are, for example, sodium stearyl fumarate, magnesium stearate, glyceryl behenate, stearic acid and adipic acid. Preferred lubricants are sodium stearyl fumarate, magnesium stearate and glyceryl behenate.

### Formulation process

The pharmaceutical compositions of the present invention are obtained by contacting Edoxaban, or a pharmaceutically acceptable salt thereof, with a water soluble vinylpyrrolidone polymer selected from the group consisting of povidone and copovidone, and a cellulose ether in a dry state or dispersed in the adequate amount of solvent) and, optionally, further inactive ingredients such as fillers and disintegrants with the proviso that sugar alcohols are excluded.

In a preferred embodiment wet granulation is used and water is used as preferred solvent. The wet granules are then dried. The dried granules are optionally sieved and blended with further ingredients, such as lubricants, and the final blend is compressed into tablets, or, alternatively, filled into capsules or sachets.

In an alternative embodiment, dry granulation is used. The mixture is then blended and compacted using appropriate compacting machine. The compacted granules or slugs are then milled and passed through appropriate sieve, blended with further ingredients, such as lubricants, and the final blend is compressed into tablets, or, alternatively, filled into capsules or sachets.

### Dosage forms

Generally, the composition of the present invention can be used as intermediate or as final dosage form. Preferably, the composition of the present invention is used as intermediate, which is preferably further processed into a dosage form. The processing into a dosage form can be achieved by means of suitable methods, such as filling into sachets or capsules or by compressing into tablets.

Therefore, the composition of the invention can be employed to prepare a dosage form, preferably an oral dosage form, more preferably a solid oral dosage form, in particular a capsule or a tablet. In a preferred embodiment, the dosage form can be a tablet, which can or cannot be film-coated. For this purpose, methods known in the art for film-coating a tablet may be employed. Generally, film-coatings can be prepared by using cellulose derivatives, poly(meth)acrylate, polyvinyl pyrrolidone, polyvinyl acetate phthalate, or natural rubbers such as carrageenan. Preferably the tablet is uncoated.

The tablet of the invention can preferably have a hardness of not less than 30 N, more preferably not less than 50 N. The hardness is determined in accordance with Ph. Eur., 6.0, Chapter 2.9.8. In addition, the tablet of the invention can preferably have a friability of less than 3%, more preferably less than 2%, in particular 0.1 to 1.2%. The friability is determined in accordance with Ph. Eur., 6.0, Chapter 2.9.7. Further, the tablet of the invention preferably can have a "content uniformity" of 93 to 107%, more preferably 95 to 105%, still more preferably 98 to 102%, particularly 99 to 101% of the average content. The "content uniformity" is determined in accordance with Ph. Eur., 6.0, Chapter 2.9.6. The above details regarding hardness, friability and content uniformity preferably relate to the non-film-coated tablet.

In a preferred embodiment the composition and/ or the dosage form according to the invention provides an immediate release of Edoxaban. This means that the release profile of the dosage forms of the invention according to USP method (paddle, 900 ml, 0.1 n HC1, 75 rpm, 37 'C) after 10 minutes usually indicates a content release of at least 75 %, preferably at least 85 %, especially at least 90 %. The release can be up to 95 %, preferably up to 100 %.

It has been unexpectedly found that the dosage form of the present invention can be administered independently from the meals of the patient, i.e. the dosage forms of the present invention are suitable to be administered before, during or after the meals. In a preferred embodiment, the dosage forms of the invention are administered once a day at a daily dose of 30 to 60 mg.

### Advantages

The dosage form of the present invention preferably shows superior and pH independent dissolution and absorption characteristics upon administration, leading to high bioavailability and desirable plasma values, like desirable AUC (area under the curve from 0 to 48 hours after oral administration), Cₘₐₓ and Tₘₐₓ values. Preferably, administration of the dosage form of the present invention results in a Tₘₐₓ value of 0.5 to 3 hours, more preferably of 0.75 to 1.5 hours. Preferably, administration of the dosage form of the present invention in a strength of 30 mg calculated for the free base of Edoxaban results in a Cₘₐₓ value of 100 to 300 ng/ml, more preferably 150 to 250 ng/ml. Preferably, administration of the dosage form of the present invention in a strength of 60 mg calculated for the free base of Edoxaban results in a Cₘₐₓ value of 150 to 500 ng/ml, more preferably 200 to 350 ng/ml. Preferably, administration of the dosage form of the present invention in a strength of 30 mg calculated for the free base of Edoxaban results in a AUC value of 500 to 3000 ng h/ml, more preferably 750 to 1500 ng h/ml. The plasma values are an average of ten single measurement values, determined upon administration to 10 male humans having a body weight of about 75 kg.

In addition, no specific fillers like sugar alcohols are needed and very good results can be obtained using common fillers like lactose, calcium phosphate and the like. Moreover high drug loads can be achieved, resulting in smaller dosage forms and improved patient compliance. Use of carbon dioxide forming agent is also avoided, which allows for cheap manufacturing and packaging. Also coating of the dosage forms can be avoided, leading to quicker and cheaper processing.

### EXAMPLES

### Examples 1-6:

| **Ingredients** | **Function** | **Amounts (mg)** | | | | | |
|---|---|---|---|---|---|---|---|
| | | **Ex. 1** | **Ex. 2** | **Ex. 3** | **Ex. 4** | **Ex. 5** | **Ex. 6** |
| Edoxaban tosylate monohydrate (corresponding to 30 mg of the free base) | Active ingredient | 40,46 | 40,46 | 40,46 | 40,46 | 40,46 | 40,46 |
| HPMC | Binder | 6 | 0 | 13 | 20 | 0 | 20 |
| HPC | Binder | 0 | 20 | 0 | 0 | 6 | 0 |
| Povidone | Binder | 6 | 0 | 13 | 6 | 20 | 0 |
| Copovidone | Binder | 0 | 20 | 0 | 0 | 0 | 6 |
| Sodium starch glycolate | Disintegrant | 16,54 | 16,54 | 16,54 | 16,54 | 16,54 | 16,54 |
| Lactose Monohydrate | Filler | 59 | 35 | 59 | 69 | 59 | 49 |
| Dibasic calcium phosphate | Filler | 64 | 40 | 50 | 40 | 30 | 50 |
| Corn starch | Filler | 0 | 20 | 0 | 0 | 20 | 10 |
| Sodium stearyl fumarate | Lubricant | 8 | 8 | 8 | 8 | 8 | 8 |
| Total | | 200 | 200 | 200 | 200 | 200 | 200 |

Edoxaban tosylate monohydrate is blended with the two binders, half of the disintegrant and with the fillers in a high shear mixer. The mixture is then granulated with an adequate amount of water using a high shear granulation. The wet granules are then dried at 50°C. The dried granules are passed through a 0.8 mm screen and blended with the rest of the disintegrant and with the lubricant using an appropriate free fall blender. The final blend is compressed into tablets using a 9 mm round punch with a target weight of 200 mg.

## Claims

1. A pharmaceutical composition comprising Edoxaban, or a pharmaceutically acceptable salt thereof, a water soluble vinylpyrrolidone polymer selected from the group consisting of povidone and copovidone, and a cellulose ether and not comprising a sugar alcohol.

2. The pharmaceutical composition of claim 1 wherein the pharmaceutically acceptable salt of Edoxaban is Edoxaban tosylate monohydrate.

3. The pharmaceutical composition of claims 1-2 wherein the water soluble vinylpyrrolidone polymer is povidone, preferably povidone K12, K15, K17, K25, K30, K60, K90 and K120.

4. The pharmaceutical composition of claims 1-2 wherein the water soluble vinylpyrrolidone polymer is copovidone, preferably copovidone is preferably selected from the group consisting of Kollidon VA 64^{R}, Plasdone S-630^{R} and Luviskol^{R} VA.

5. The pharmaceutical composition of claims 1-4 wherein the cellulose ether is selected from the group consisting of hydroxypropyl methylcellulose, hydroxypropyl cellulose, ethylcellulose, hydroxyethyl cellulose, carboxymethyl cellulose and methylcellulose.

6. The pharmaceutical composition of claims 1-5 wherein the cellulose ether is hydroxypropyl methylcellulose.

7. The pharmaceutical composition of claims 1-6 further comprising one or more active ingredients selected from the group consisting of fillers, disintegrants and lubricants.

8. A dosage form comprising the pharmaceutical composition of claims 1-7.

9. The dosage form of claim 8, which is a tablet.

10. The dosage form of claims 8-9, which is an uncoated tablet.

11. A process for the preparation of the pharmaceutical composition of claims 1-7 comprising granulating Edoxaban, or a pharmaceutically acceptable salt thereof, a water soluble vinylpyrrolidone polymer selected from the group consisting of povidone and copovidone, and a cellulose ether.

12. The process of claim 11, wherein wet granulation is used.

13. A pharmaceutical composition obtainable by the process of claims 11-12.

14. The pharmaceutical composition of claims 1-7 or 13 or the dosage form of claims 8-10 for use as a medicament.

15. The pharmaceutical composition of claims 1-7 or 13 the dosage form of claims 8-10 for use in preventing and/or treating thrombosis or embolism.

## Patentansprüche

1. Pharmazeutische Zusammensetzung umfassend Edoxaban, oder ein pharmazeutisch akzeptables Salz davon, ein wasserlösliches Vinylpyrrolidon-Polymer ausgewählt aus der Gruppe bestehend aus Povidon und Copovidon, und ein Celluloseether und nicht umfassend einen Zuckeralkohol.

2. Pharmazeutische Zusammensetzung von Anspruch 1, wobei das pharmazeutisch akzeptable Edoxaban-Salz Edoxaban-Tosylatmonohydrat ist.

3. Pharmazeutische Zusammensetzung der Ansprüche 1 - 2, wobei das wasserlösliche Vinylpyrrolidon-Polymer Povidon ist, bevorzugt Povidon K12, K15, K17, K25, K30, K60, K90 und K120.

4. Pharmazeutische Zusammensetzung der Ansprüche 1 - 2, wobei das wasserlösliche Vinylpyrrolidon-Polymer Copovidon ist, bevorzugt ist Copovidon bevorzugt ausgewählt aus der Gruppe bestehend aus Kollidon VA 64^{R}, Plasdon S-630^{R} und Luviskol^{R} VA.

5. Pharmazeutische Zusammensetzung der Ansprüche 1 - 4, wobei der Celluloseether ausgewählt ist aus der Gruppe bestehend aus Hydroxypropylmethylcellulose, Hydroxypropylcellulose, Ethylcellulose, Hydroxyethylcellulose, Carboxymethylcellulose und Methylcellulose.

6. Pharmazeutische Zusammensetzung der Ansprüche 1 - 5, wobei der Celluloseether Hydroxypropylmethylcellulose ist.

7. Pharmazeutische Zusammensetzung der Ansprüche 1 - 6, ferner umfassend ein oder mehrere Wirkstoffe ausgewählt aus der Gruppe bestehend aus Füllstoffen, Sprengmitteln und Gleitmitteln.

8. Darreichungsform umfassend die pharmazeutische Zusammensetzung der Ansprüche 1 - 7.

9. Darreichungsform des Anspruchs 8, welche eine Tablette ist.

10. Darreichungsform der Ansprüche 8 - 9, welche eine unbeschichtete Tablette ist.

11. Verfahren zur Herstellung der pharmazeutischen Zusammensetzung der Ansprüche 1 - 7, umfassend Granulieren von Edoxaban, oder eines pharmazeutisch akzeptablen Salzes davon, eines wasserlöslichen Vinylpyrrolidon-Polymers ausgewählt aus der Gruppe bestehend aus Povidon und Copovidon, und eines Celluloseethers.

12. Verfahren gemäß Anspruch 11, wobei Feuchtgranulierung verwendet wird.

13. Pharmazeutische Zusammensetzung erhältlich durch das Verfahren der Ansprüche 11 - 12.

14. Pharmazeutische Zusammensetzung der Ansprüche 1 - 7 oder 13 oder Darreichungsform der Ansprüche 8 - 10, zur Verwendung als ein Medikament.

15. Pharmazeutische Zusammensetzung der Ansprüche 1 - 7 oder 13, Darreichungsform der Ansprüche 8 - 10, zur Verwendung bei der Prävention und/oder Behandlung von Thrombose oder Embolie.

## Revendications

1. Composition pharmaceutique comprenant de l'Edoxaban, ou un de ses sels pharmaceutiquement acceptables, un polymère de vinylpyrrolidone hydrosoluble choisi dans le groupe constitué par la povidone et la copovidone, et un éther de cellulose et ne comprenant pas d'alcool de sucre.

2. Composition pharmaceutique selon la revendication 1, dans laquelle le sel pharmaceutiquement acceptable de l'Edoxaban est le tosylate monohydrate d'Edoxaban.

3. Composition pharmaceutique selon les revendications 1 - 2 dans laquelle le polymère de vinylpyrrolidone hydrosoluble est la povidone, de préférence la povidone K12, K15, K17, K25, K30, K60, K90 et K120.

4. Composition pharmaceutique selon les revendications 1-2 dans laquelle le polymère de vinylpyrrolidone hydrosoluble est la copovidone, de préférence la copovidone est de préférence choisie dans le groupe constitué par le Kollidon VA 64^{R}, la Plasdone S-630^{R} et le Luviskol^{R} VA.

5. Composition pharmaceutique selon les revendications 1-4 dans laquelle l'éther de cellulose est choisi dans le groupe constitué par l'hydroxypropyl méthylcellulose, l'hydroxypropylcellulose, l'éthylcellulose, l'hydroxyéthylcellulose, la carboxyméthylcellulose et la méthylcellulose.

6. Composition pharmaceutique selon les revendications 1-5 dans laquelle l'éther de cellulose est l'hydroxypropyl méthylcellulose.

7. Composition pharmaceutique selon les revendications 1-6 comprenant en outre un ou plusieurs ingrédients actifs choisis dans le groupe constitué de charges, de délitants et de lubrifiants.

8. Forme posologique comprenant la composition pharmaceutique selon les revendications 1-7.

9. Forme posologique selon la revendication 8 qui est un comprimé.

10. Forme posologique selon les revendications 8-9 qui est un comprimé non enrobé.

11. Procédé de préparation de la composition pharmaceutique selon les revendications 1-7 comprenant la granulation de l'Edoxaban, ou d'un de ses sels pharmaceutiquement acceptables, d'un polymère de vinylpyrrolidone hydrosoluble choisi dans le groupe consistant en la povidone et la copovidone et d'un éther de cellulose.

12. Procédé selon la revendication 11, dans lequel une granulation humide est utilisée.

13. Composition pharmaceutique pouvant être obtenue par le procédé selon les revendications 11-12.

14. Composition pharmaceutique selon les revendications 1-7 ou 13 ou la forme posologique des revendications 8 - 10 pour une utilisation comme médicament.

15. Composition pharmaceutique selon les revendications 1-7 ou 13, la forme galénique des revendications 8-10 pour une utilisation pour prévenir et/ou traiter une thrombose ou une embolie.
